# EUROPEAN PATENT SPECIFICATION

(11) **EP 2 042 609 B1**
(45) Date of publication and mention of the grant of the patent: **22.12.2010**
(21) Application number: 08016874.3
(22) Date of filing: 25.09.2008
(51) Int. Cl.: C12Q 1/00, G01N 33/543, G01N 27/327

(54) **Biosensor**
Biosensor
Biocapteur

(30) Priority: 26.09.2007 JP 2007248500
(43) Date of publication of application: 01.04.2009
(73) Proprietor: FUJIFILM Corporation, Tokyo 106-0031 (JP)
(72) Inventor: Kuruma, Koji, Tokyo 106-0031 (JP); Minami, Koichi, Ashigarakami-gun Kanagawa 258-8577 (JP)
(74) Representative: HOFFMANN EITLE

(56) References cited:
- WO-A-99/56119
- WO-A-02/052260
- WO-A-2005/017122
- JP-A- 3 054 447
- LOEFAAS S ET AL: "A NOVEL HYDROGEL MATRIX ON GOLD SURFACES IN SURFACE PLASMON RESONANCE SENSORS FOR FAST AND EFFICIENT COVALENT IMMOBILIZATION OF LIGANDS" JOURNAL OF THE CHEMICAL SOCIETY, CHEMICAL COMMUNICATIONS, CHEMICAL SOCIETY. LETCHWORTH, GB, no. 21, 1 January 1990 (1990-01-01), pages 1526-1528, XP008050238 ISSN: 0022-4936
- O'SHANNESSY D J ET AL: "Immobilization chemistries suitable for use in the BIAcore surface plasmon resonance detector" ANALYTICAL BIOCHEMISTRY, ACADEMIC PRESS INC. NEW YORK, vol. 205, no. 1, 15 August 1992 (1992-08-15), pages 132-136, XP024818761 ISSN: 0003-2697 [retrieved on 1992-08-15]

## Description

### Technical Field

The present invention relates to a biosensor having excellent preservation stability.

### Background Art

Recently, a large number of measurements using intermolecular interactions such as immune responses are being carried out in clinical tests, etc. However, since conventional methods require complicated operations or labeling substances, several techniques are used that are capable of detecting the change in the binding amount of a test substance with high sensitivity without using such labeling substances. Examples of such a technique may include a surface plasmon resonance (SPR) measurement technique, a quartz crystal microbalance (QCM) measurement technique, and a measurement technique of using functional surfaces ranging from gold colloid particles to ultra-fine particles. The SPR measurement technique is a method of measuring changes in the refractive index near an organic functional film attached to the metal film of a chip by measuring a peak shift in the wavelength of reflected light, or changes in amounts of reflected light in a certain wavelength, so as to detect adsorption and desorption occurring near the surface. The QCM measurement technique is a technique of detecting adsorbed or desorbed mass at the ng level, using a change in frequency of a crystal due to adsorption or desorption of a substance on gold electrodes of a quartz crystal (device). In addition, the ultra-fine particle surface (nm level) of gold is functionalized, and physiologically active substances are immobilized thereon. Thus, a reaction to recognize specificity among physiologically active substances is carried out, thereby detecting a substance associated with a living organism from sedimentation of gold fine particles or sequences.
In all of the above-described techniques, the surface where a physiologically active substance is immobilized is important, since a specific binding reaction between the physiologically active substance and a test substance is measured to analyze interactions of biomolecules.
In general, as a detection surface having a functional group capable of immobilizing a physiologically active substance thereon, Japanese Patent No. 2815120 discloses in detail a method for producing a hydrogeL Specifically, a compound having a mercapto group and a straight-chain alkyl is bound to a gold film in a layer state, so as to form a barrier layer. Further, the functional group of the aforementioned compound is used to bind a water-soluble polymer, so as to increase the amount of a physiologically active substance immobilized. However, this production method has been problematic in that the mercapto group is oxidized by oxygen and in that the barrier layer or the water-soluble polymer layer is thereby dissociated from the gold film. This phenomenon occurs frequently as the length of the alkyl group becomes short.

Löfås et al., J. Chem. Soc., Chem. Commun. (1990) No. 21, pp. 1526-1528, discloses surface plasmon resonance sensors for biospecific interaction analysis, in which a glass substrate coated with a gold film is treated with a self-assembled monolayer of 16-mercaptohexadecan-1-ol and epichlorohydrin, subsequently with dextran.

### Disclosure of the Invention

It is an object of the present invention to solve the aforementioned problem of the prior art technique. In other words, it is an object of the present invention to provide a biosensor having excellent preservation stability. Specifically, it is an object of the present invention to provide a biosensor, in which dissociation of a hydrophilic polymer from a metal substrate after preservation for a certain period of time is suppressed.
As a result of intensive studies, the present inventors have found that the aforementioned problem can be solved by setting the total film thickness of a first hydrophilic polymer layer and a second hydrophilic polymer layer in a dry state between 20 nm and 100 nm in a biosensor wherein the first hydrophilic polymer layer is immobilized on a self-assembled membrane applied onto the surface of a metal substrate, and at least one second hydrophilic polymer layer is applied onto the first hydrophilic polymer layer. Thus, the present invention has been completed.
The present invention provides a biosensor wherein a first hydrophilic polymer layer is immobilized on a self-assembled membrane applied onto the surface of a metal substrate, a second hydrophilic polymer layer is applied onto the first hydrophilic polymer layer, and the total film thickness of the first hydrophilic polymer layer and the second hydrophilic polymer layer in a dry state is between 20 nm and 100 nm.
Preferably, the hydrophilic polymer that forms the first hydrophilic polymer layer and the second hydrophilic polymer layer is a polysaccharide.
Preferably, the self-assembled membrane is formed by a compound having a mercapto group.
Preferably, the self-assembled membrane is formed by a compound represented by the following formula A-1:

**HS(CH₂)ₙX** A-1

wherein n represents an integer between 3 and 20, and X represents a functional group for binding a hydrophilic polymer.
Preferably, the self-assembled membrane is formed by coating the surface of the metal substrate with an aqueous solution of a compound having a straight-chain alkyl group containing 4 to 10 carbon atoms.
Preferably, the biosensor of the present invention is used in nonelectrochemical detection.
Preferably, the biosensor of the present invention is used in surface plasmon resonance analysis.
According to the present invention, it became possible to provide a biosensor with good preservative quality, in which dissociation of a hydrophilic polymer from a metal substrate after preservation for a certain period of time is suppressed.

### Brief Description of the Invention

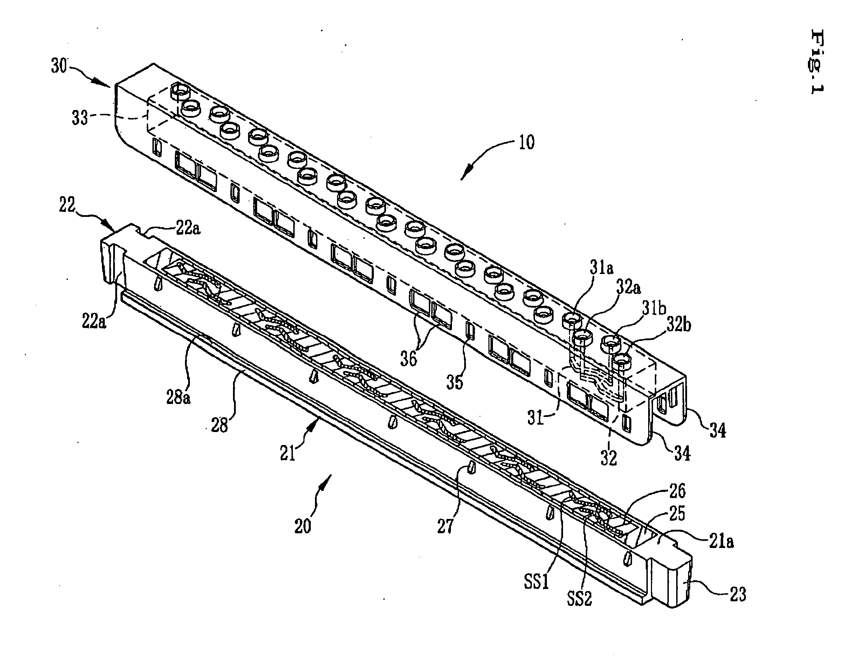

Figure 1 shows an exploded perspective view of a sensor unit

### Best Mode for Carrying Out the Invention

The embodiments of the present invention will be described in detail below.
The biosensor of the present invention is a biosensor wherein a first hydrophilic polymer layer is immobilized on a self-assembled membrane applied onto the surface of a metal substrate, a second hydrophilic polymer layer is applied onto the first hydrophilic polymer layer, and the total film thickness of the first hydrophilic polymer layer and the second hydrophilic polymer layer in a dry state is between 20 nm and 100 nm.
In the present invention, the total film thickness of a first hydrophilic polymer layer and a second hydrophilic polymer layer in a dry state is between 20 nm and 100 nm, and more preferably between 20 nm and 50 nm. In the present invention, by setting the film thickness within the aforementioned range, the residual ratio of the first hydrophilic polymer is kept high even after a certain period of time has passed, and thus, an effect of immobilizing a large amount of physiologically active substance can be achieved. The number of the second hydrophilic polymer layers is not particularly limited, as long as it is at least a single layer. Thus, two or more layers may also be applied herein, but a single layer is preferable. The aforementioned effect can be achieved even using only the first hydrophilic polymer layer and by setting the film thickness in a dry state between 20 nm and 100 nm.
In the present invention, the expression "the film thickness in a dry state" is used to mean "the film thickness obtained after leaving at rest at a 5°C or more difference between dry- and wet-bulb temperatures for 30 minutes or more." Herein, a difference between dry- and wet-bulb temperatures is preferably 7°C or more, and most preferably 10°C or more.
A method for measuring the film thickness of a hydrophilic polymer layer in a dry state is not particularly limited. Such a film thickness can be evaluated by AFM or ellipsometry, for example. Specifically, as a measurement method using AFM, the relative value of an applied portion/an unapplied portion at a tapping mode is defined as a film thickness, and measurement can be carried out. On the other hand, in ellipsometry, a change in the relative phase of the reflected polarized beam is measured, so as to calculate the film thickness.
The biosensor in the present invention has as broad a meaning as possible, and the term biosensor is used herein to mean a sensor, which converts an interaction between biomolecules into a signal such as an electric signal, so as to measure or detect a target substance. The conventional biosensor is comprised of a receptor site for recognizing a chemical substance as a detection target and a transducer site for converting a physical change or chemical change generated at the site into an electric signal. In a living body, there exist substances having an affinity with each other, such as enzyme/substrate, enzyme/coenzyme, antigen/antibody, or hormone/receptor. The biosensor operates on the principle that a substance having an affinity with another substance is immobilized on a substrate to be used as a molecule-recognizing substance, so that the corresponding substance can be selectively measured.
As the metal substrate in the present invention, a metal or a metal film placed on a carrier can be used. A metal constituting the metal surface or metal film is not particularly limited, as long as surface plasmon resonance is generated when the metal is used for a surface plasmon resonance biosensor. Examples of a preferred metal may include free-electron metals such as gold, silver, copper, aluminum or platinum. Of these, gold is particularly preferable. These metals can be used singly or in combination. Moreover, considering adherability to the above carrier, an interstitial layer consisting of chrome or the like may be provided between the carrier and a metal layer.
The film thickness of a metal film is not limited. When the metal film is used for a surface plasmon resonance biosensor, the thickness is preferably between 0.1 nm and 500 nm, and particularly preferably between 1 nm and 200 nm. If the thickness exceeds 500 nm, the surface plasmon phenomenon of a medium cannot be sufficiently detected. Moreover, when an interstitial layer consisting of chrome or the like is provided, the thickness of the interstitial layer is preferably between 0.1 nm and 10 nm.
Formation of a metal film may be carried out by common methods, and examples of such a method may include sputtering method, evaporation method, ion plating method, electroplating method, and nonelectrolytic plating method.
When a substrate of the present invention is used for a surface plasmon resonance biosensor, examples of a substrate may include, generally, optical glasses such as BK7, and synthetic resins. More specifically, materials transparent to laser beams, such as polymethyl methacrylate, polyethylene terephthalate, polycarbonate or a cycloolefin polymer, can be used. For such a substrate, materials that are not anisotropic with regard to polarized light and have excellent workability are preferably used. The description "placed on a carrier" is used herein to mean a case where a metal film is placed on a carrier such that it directly comes into contact with the carrier, as well as a case where a metal film is placed via another layer without directly coming into contact with the carrier. Preferably, the refractive index of a material for the carrier is between 1.4 and 1.7. When an affinity between a physiologically active substance in aqueous solution and a compound is measured by using surface plasmon resonance, a dark line by resonance can not be obtained as reflected light if the refractive index of the substrate is low. Also, incidence angle and reflection angle of light source to the substrate must be low if the refractive index is high, and it may make it difficult to construct the device.
The aforementioned substrate is immobilized on the dielectric block of a sensor unit and is unified therewith to construct a measurement chip. This measurement chip may be exchangeably formed. An example will be given below.
Figure 1 is an exploded perspective view of a sensor unit 10 used in a measurement that utilizes SPR. The sensor unit 10 is composed of a total internal reflection prism (optical block) 20 that is a transparent dielectric body and a flow channel member 30 equipped on the total internal reflection prism 20. The flow channel member 30 has two types of flow channels, namely, a first flow channel 31 located on the back side of the figure and a second flow channel 32 located on the front side of the figure. When the sensor unit 10 is used in measurement, the two types of flow channels 31 and 32 are used in combination to measure a single sample. However, the details will be described later. In the flow channel member 30, six flow channels 31 and six flow channels 32 are established in the longitudinal direction, so that six samples can be measured in a single sensor unit 10. It is to be noted that the number of either the flow channel 31 or 32 is not limited to six, but that it may be 5 or less, or 7 or more.
The total internal reflection prism 20 is composed of a prism main body 21 formed in a long trapezoidal shape, a gripper 22 established at one end of the prism main body 21, and a projecting portion 23 established at the other end of the prism main body 21. This total internal reflection prism 20 is molded by extrusion molding, for example. The prism main body 21, the gripper 22, and the projecting portion 23 are integrally molded.
The prism main body 21 has a substantially trapezoidal longitudinal section wherein the lower base is longer than the upper base. Light irradiated from the lateral side of the bottom is gathered to an upper surface 21a. A metal film (thin film layer) 25 for exciting SPR is established on the upper surface 21a of the prism main body 21. The shape of the metal film 25 is rectangular such that it faces the flow channels 31 and 32 of the flow channel member 30. The metal film 25 is molded by an evaporation method, for example. The Metal film 25 is made of gold, silver, or the like, and the thickness thereof is 50 nm, for example. The thickness of the metal film 25 is selected as appropriate, depending on the material of the metal film 25, the wavelength of light irradiated during the measurement, etc.
On the metal film 25, a polymer layer 26 is established. The polymer layer 26 has a binding group for immobilizing a physiologically active substance. A physiologically active substance is immobilized on the metal film 25 via the polymer layer 26.
In the present invention, a metal film is coated with a self-assembled membrane-forming molecule (hereinafter referred to as a "step of coating with a self-assembled membrane-forming molecule," as appropriate), and thereafter, a hydrophilic polymer comprising an active esterified carboxyl group (hereinafter referred to as a "step of activating a hydrophilic polymer") is allowed to react with the aforementioned organic layer, so as to establish a hydrophilic polymer layer capable of immobilizing a physiologically active substance on a substrate (hereinafter referred to as a "step of establishing a hydrophilic polymer," as appropriate). In the present invention, a physiologically active substance is immobilized on the thus obtained hydrophilic polymer layer on the substrate, as necessary (hereinafter referred to as a "step of immobilizing a physiologically active substance," as appropriate). Thereafter, a second hydrophilic polymer is further added thereto (hereinafter referred to as a "step of adding a stabilizer," as appropriate), so as to obtain a physiologically active substance-immobilized substrate having excellent preservation stability.

### <Step of coating with a self-assembled membrane-forming molecule>

In the present invention, the sell-assembled membrane-forming molecule has a role for connecting a metal film with a first hydrophilic polymer layer. Hereafter, self-assembled membranes (SAMs) having self-assembled membrane-forming molecules will be described.
A method for coating a metal film with the use of a self-assembled membrane (SAMs) has been actively developed by Professor Whitesides et al. (Harvard University). Details of the method are reported in, for example, Chemical Review, 105, 1103-1169 (2005). When gold is used as a metal, an orientational self-assembled monomolecular film is formed with the use of an alkanethiol derivative represented by the following formula A-1 (wherein n represents an integer from 3 to 20, and X represents a functional group) based on the van der Waals force between an Au-S bond and an alkyl chain. A self-assembled membrane is formed by a very simple method, wherein a gold substrate is immersed in a solution of an alkanethiol derivative. In the present invention, it is preferred that the compound has an amino group at its terminal. A self-assembled membrane is formed with the use of a compound (represented by the following formula A-1 where X is NH₂) so that it becomes possible to coat a gold surface with an organic layer comprising an amino group:

**HS(CH₂)ₙX** A-1

An alkanethiol having an amino group at the end may be a compound comprising a thiol group and an amino group linked via an alkyl chain (formula A-2) (in the formula A-2, n represents an integer of 3 to 20), or may be a compound obtained by reaction between alkanethiol having a carboxyl group at the end (formula A-3 or A-4) (in the formula A-3, n represents an integer of 3 to 20, and in the formula A-4, n each independently represents an integer of 1 to 20) and a large excess of hydrazide or diamine. The reaction between alkanethiol having a carboxyl group at the end and a large excess of hydrazide or diamine may be performed in a solution state. Alternatively, the alkanethiol having a carboxyl group at the end may be bound to the substrate surface and then reacted with a large excess of hydrazide or diamine.

HS(CH₂)ₙNH₂ A-2

HS(CH₂)ₙCOOH A-3

HS(CH₂)ₙ(OCH₂CH₂)ₘOCH₂COOH A-4

The repeating number of alkyl group of the formulas A-2 to A-4 is preferably 3 to 20, more preferably 3 to 16, and most preferably 4 to 8. If the alkyl chain is short, formation of self-assembled membrane becomes difficult, and if the alkyl chain is long, water solubility decreases and the handling becomes difficult. The repeating number (m) of ethyleneoxide group is independently an integer of 1 to 20, preferably an integer of 3 to 20, more preferably an integer of 3 to 16, and most preferably an integer of 4 to 8.
Any compound may be used as the diamine used in the present invention. An aqueous diamine is preferable for use in the biosensor surface. Specific examples of the aqueous diamine may include aliphatic diamine such as ethylenediamine, tetraethylenediamine, octamethylenediamine, decamethylenediamine, piperazine, triethylenediamine, diethylenetriamine, triethylenetetraamine, dihexamethylenetriamine, and 1,4-diaminocyclohexane, and aromatic diamine such as paraphenylenediamine, metaphenylenediamine, paraxylylenediamine, metaxylylenediamine, 4,4'-diaminobiphenyl, 4,4'-diaminodiphenylmethane, 4,4'-diaminodiphenylketone, and 4,4'-diaminodiphenylsulfonic acid. From the viewpoint of increasing the hydrophilicity of the biosensor surface, a compound comprising two amino groups linked via an ethylene glycol unit (formula A-5) may also be used. The diamine used in the present invention is preferably ethylenediamine or the compound represented by the formula A-5 (in the formula A-5, n and m each independently represent an integer of 1 to 20), more preferably ethylenediamine or 1,2-bis(aminoethoxy)ethane (represented by the formula A-5 wherein n=2 and m=1).

**H₂N(CH₂)ₙ(OCH₂CH₂)ₘO(CH₂)ₙNH₂** A-5

The alkanethiol having an amino group may form a self-assembled membrane by itself or may form a self-assembled membrane by mixing it with another alkanethiol. It is preferred for use in the biosensor surface that a compound capable of suppressing the nonspecific adsorption of a physiologically active substance should be used as the another alkanethiol. The aforementioned Professor Whitesides et al. have investigated in detail self-assembled membrane capable of suppressing the nonspecific adsorption of a physiologically active substance and have reported that a self-assembled membrane formed from alkanethiol having a hydrophilic group is effective for suppressing nonspecific adsorption (Langmuir, 17, 2841-2850, 5605-5620, and 6336-6343 (2001)). In the present invention, any of compounds described in the aforementioned papers may be used preferably as the alkanethiol that forms a mixed monolayer with an alkanethiol having an amino group. In terms of excellent ability to suppress nonspecific adsorption and ease of acquisition, it is preferred that alkanethiol having a hydroxyl group (formula A-6) or alkanethiol having an ethylene glycol unit (formula A-7) (in the formula A-6, n represents an integer of 3 to 20, and in the formula A-7, n and m each independently represent an integer of 1 to 20) should be used as the alkanethiol that forms a mixed monolayer with an alkanethiol having an amino group.

**HS(CH₂)ₙOH** A-6

**HS(CH₂)ₙ(OCH₂CH₂)ₘOH** A-7

When alkane thiol having an amino group is mixed with another alkane thiol to form a self-assembled membrane, the repeating number of alkyl group of the formulas A-2 to A-4 is preferably 4 to 20, more preferably 4 to 16, and most preferably 4 to 10. Further, the repeating number of alkyl group of the formulas A-6 and A-7 is preferably 3 to 16, more preferably 3 to 12, and most preferably 3 to 8.
In the present invention, it is possible to mix alkanethiol having an amino group and alkanethiol having a hydrophilic group at an arbitrary ratio. However, when the content of alkanethiol having an amino group is low, the amount of actively esterified carboxyl group-containing polymer to be bound decreases. When the content of alkanethiol having a hydrophilic group is low, the capacity for suppression of nonspecific adsorption is reduced. Thus, the mixing ratio of alkanethiol having an amino group to alkanethiol having a hydrophilic group is preferably 1:1 to 1:1,000,000, more preferably 1:1 to 1:1,000, and further preferably 1:1 to 1:10. In view of reduction of steric hindrance upon a reaction with an actively esterified carboxyl group-containing polymer, the molecular length of alkanethiol having an amino group is preferably longer than that of alkanethiol having a hydrophilic group.
As alkanethiol used for the present invention, compounds synthesized based on Abstract, Curr. Org. Chem., 8, 1763-1797 (2004) (Professor Graybowski, Northwestern University) and references cited therein or a commercially available compound may be used. It is possible to purchase such compounds from Dojindo Laboratories, Aldrich, SensoPath Technologies, Frontier Scientific Inc., and the like. In the present invention, disulfide compounds that are oxidation products of alkanethiol can be used in the same manner as alkanethiol.
The aforementioned self-assembled membcane-forming molecule can be dissolved in an organic solvent (e.g. ethanol, etc.), water, or a mixed solvent of such an organic solvent and water, before use. Preferably, the aforementioned self-assembled membrane-forming molecule can be dissolved in water before use.

### <Step of activating hydrophilic polymer that forms first hydrophilic polymer layer>

As a hydrophilic polymer that forms a first hydrophilic polymer layer immobilized on a self-assembled membrane applied onto the surface of a metal substrate, a carboxyl group-containing synthetic polymer and a carboxyl group-containing polysaccharide can be used. Examples of such a carboxyl group-containing synthetic polymer include polyacrylic acid, polymethacrylic acid, and their copolymers such as a methacrylic acid copolymer, an acrylic acid copolymer, an itaconic acid copolymer, a crotonic acid copolymer, a maleic acid copolymer, a partially esterified maleic acid copolymer or a product formed by adding an acid anhydride to a polymer having a hydroxyl group, which are described, for example, in line 2, upper right column, page 3 to line 9, lower left column, page 6 of JP Patent Publication (Kokai) No. 59-53836 A (1984), and in line 1, lower left column, page 3 to line 3, upper left column, page 5 of JP Patent Publication (Kokai) No. 59-71048 A (1984). A carboxyl group-containing polysaccharide may be any one of an extract from a natural plant, a microbial fermentation product, a product synthesized by an enzyme, and a chemically synthesized product. Specific examples of such a polysaccharide include hyaluronic acid, chondroitin sulfate, heparin, dermatan sulfate, carboxymethylcellulose, carboxyethylcellulose, cellouronic acid, carboxymethyl chitin, carboxymethyl dextran, and carboxymethyl starch. As a carboxyl group-containing polysaccharide, a commercially available compound can be used. Specific examples of such a commercially available compound include CMD, CMD-L and CMD-D40 (carboxymethyl dextran products manufactured by Meito Sangyo Co., Ltd.), carboxymethylcellulose sodium (manufactured by Wako Pure Chemical Industries, Ltd.), and sodium alginate (manufactured by Wako Pure Chemical Industries, Ltd.).
A hydrophilic polymer that forms the first hydrophilic polymer layer is preferably a polysaccharide containing a carboxyl group, and more preferably carboxymethyl dextran.
The molecular weight of the polymer containing a carboxyl group used in the present invention is not particularly limited. The mean molecular weight is preferably between 1,000 and 5,000,000, and more preferably between 10,000 and 2,000,000. If the mean molecular weight is smaller than the aforementioned range, the amount of a physiologically active substance immobilized becomes small. If the mean molecular weight is larger than the aforementioned range, the solution viscosity becomes high, so that it becomes difficult to handle it.

A known technique can be preferably used as a method for activating polymers containing carboxyl groups. Preferred examples of such method include: a method that involves activating carboxyl groups using 1-(3-Dimethylaminopropyl)-3 ethylcarbodiimide (EDC) (waler-soluble carbodiimide) alone, or using EDC and N-Hydroxysuccinimide (NHS). It becomes possible to produce the substrate of the present invention by causing a polymer containing a carboxyl group that has been activated by these techniques to react with a substrate having an amino group.
Moreover, another method for activation of a polymer containing a carboxyl groups is a method that uses a nitrogen-containing compound. Specifically, a nitrogen-containing compound represented by the following formula (Ia) of (Ib) [wherein R₁ and R₂ mutually independently denote a carbonyl group, a carbon atom, or a nitrogen atom, which may have a substituent, R₁ and R₂ may form 5- to 6-membered rings via binding, "A" denotes a carbon atom or a phosphorus atom, which has a substituent, "M" denotes an (n-1)-valent element, and "X" denotes a halogen atom] can also be used.

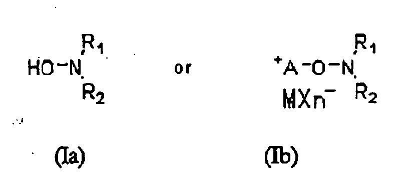

R₁ and R₂ mutually independently denote a carbonyl group, a carbon atom, or a nitrogen atom, which may have a substituent, and preferably R₁ and R₂ form 5- to 6-membered rings via binding. Particularly preferably, hydroxysuccinic acid, hydroxyphthalic acid, 1-hydroxybenzotriazole, 3,4-dihydroxy-3-hydsoxy-4-oxo-1,2,3-benzotriazine, and a derivative thereof are provided.
Further preferably, a nitrogen-containing compound represented by the following compound can also be used.

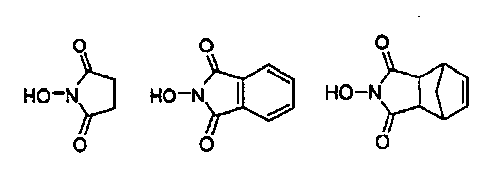

More preferably, a compound represented by the following formula (II) [wherein "Y" and "Z" mutually independently denote CH or a nitrogen atom] can also be used as a nitrogen-containing compound.

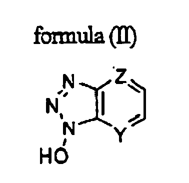

Specifically, the following compounds can be used, for example.

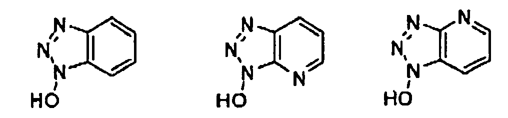

Further preferably, the following compound can also be used as a nitrogen-containing compound.

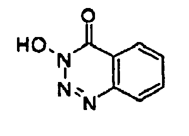

Further preferably, a compound represented by the following formula (III) [wherein "A" denotes a carbon atom or a phosphorus atom, which has a substituent, "Y" and "Z" mutually independently denote CH or a nitrogen atom, "M" denotes a (n-1)-valent element, and X denotes a halogen atom] can also be used as a nitrogen-containing compound.

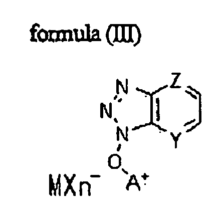

A substituent of a carbon atom or a phosphorus atom denoted by "A" is preferably an amino group having a substituent. Furthermore, a dialkylamino group such as a dimethylamino group or a pyrrolidino group is preferable. Examples of an (n-1)-valent element denoted by "M" include a phosphorus atom, a boron atom, and an arsenic atom. A preferable example of such (n-1)-valent element is a phosphorus atom. Examples of a halogen atom denoted by "X" include a fluorine atom, a chlorine atom, a bromine atom, and an iodine atom. A preferable example of such halogen atom is a fluorine atom.

Furthermore, specific examples of such nitrogen-containing compound represented by formula (III) include the following compounds, for example.

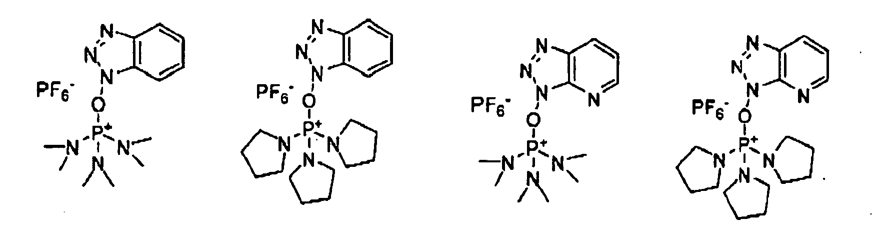

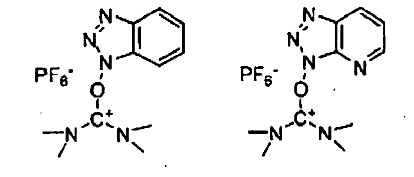

Further preferably, a compound represented by the following formula (IV) [wherein "A" denotes a carbon atom or a phosphorus atom, which has a substituent, "M" denotes an (n-1)-valent element, and "X" denotes a halogen atom] can also be used as a nitrogen-containing compound.

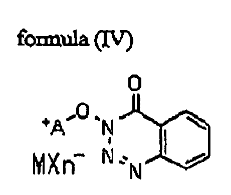

Specifically, the following compound can be used, for example.

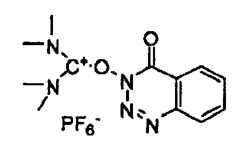

The mixing ratio of the above nitrogen compound as an activating agent may be any amount which is generally used in this purpose of use. In view of immobilizing the polymer sufficiently, it is preferred that the mixing molar ratio of the nitrogen compound to a functional group (for example, carboxyl group) in the polymer layer 26 is 1 x 10 ⁻⁷ to 1.
In addition, from the viewpoint of immobilization of a sufficient amount of polymer, a phenol derivative preferably has an electron attracting group. Further, from the viewpoint of immobilization of a sufficient amount of polymer, the σ value of such an electron attracting group is preferably 0.3 or greater. Specifically, the following compounds (IV-1 to IV-4) can be used, for example. In (VI-4), X⁻ represents anion.
The mixing ratio of the phenol derivative as an activating agent may be any amount which is generally used in this purpose of use. In view of immobilizing the polymer sufficiently, it is preferred that the mixing molar ratio of the phenol derivative to a functional group (for example, carboxyl group) in the polymer layer 26 is 1 x 10⁻⁴ to 1.

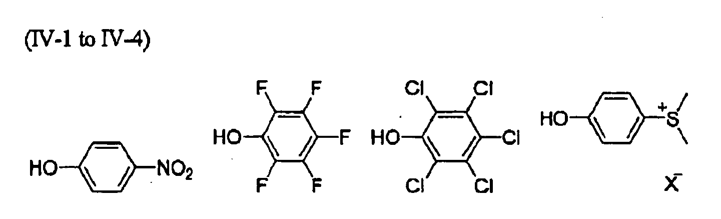

Furthermore, a carbodiimide derivative can further be used separately for such method for activating a polymer containing carboxyl group in combination with the above compounds. Preferably, a water-soluble carbodiimide derivative can be used in combination with such compounds. Further preferably, the following compound (1-Ethyl-3-(3-dimethylaminogropyl)carbodiimide, hydrochloride) can be used in combination with such compounds.

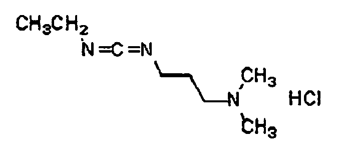

The above carbodiimide derivative and nitrogen-containing compound or phenol derivative can be used not only in such manner, but also independently, if desired. Preferably, a carbodiimide derivative and a nitrogen-containing compound are used in combination.
Furthermore, the morpholine derivative can be used can also be used in such method for activating carboxyl groups. The compound can be used independently and can also be used in combination with a carbodiimide derivative, a nitrogen-containing compound, and/or a phenol derivative.

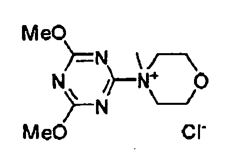

### <Step of forming first hydrophilic polymer layer>

In the present invention, a hydrophilic polymer that forms a first hydrophilic polymer layer is preferably a polymer containing an active esterified carboxyl group. Such a polymer containing an active esterified carboxyl group may be allowed to react with a substrate in the form of a solution.
Otherwise, a thin film of the polymer is formed on a substrate by applying a method such as spin-coating, so that the polymer may be allowed to react with the substrate. The reaction is preferably earned out in a state where such a thin film is formed.
The film thickness of the first hydrophilic polymer layer in a dry state (dry conditions are as described above in the present specification) is preferably between 0.1 and 100 nm, more preferably between 0.5 and 50 nm, and particularly preferably between 1 and 30 nm.
As stated above, the polymer containing an actively esterified carboxyl group in the present invention may be preferably allowed to react with a substrate in the state of a thin film. As a method for forming a thin film on a substrate, known methods can be used. Specific examples of such methods that can be used include an extrusion coating method, a curtain coating method, a casting method, a screen printing method, a spin coating method, a spray coating method, a slide bead coating method, a slit and spin method, a slit coating method, a dye coating method, a dip coating method, a knife coating method, a blade coating method, a flow coating method, a roll coating method, a wire-bar coating method, and a transfer printing method. These methods for forming a thin film are described in "Progress in Coating Technology (Coating Gijutsu no Shinpo)" written by Yuji Harazaki, Sogo Gijutsu Center (1988); "Coating Technology (Coating Gijutsu)" Technical Information Institute Co., Ltd. (1999); "Aqueous Coating Technology (Suisei Coating no Gijutsu)" CMC (2001); "Evolving Organic Thin Film: Edition for Deposition (Shinka-suru Organic Thin Film: Seimaku hen)" Sumibe Techno Research Co., Ltd. (2004); "Polymer Surface Processing Technology (Polymer Hyomen Kako Gaku)" written by Akira Iwamori, Gihodo Shuppan Co., Ltd. (2005); and the like. As the method for forming a thin film on a substrate of the present invention, a spray coating method or a spin coating method is preferable. Further, a spin coating method is more preferable. This is because it allows a coating film having a controlled film thickness to be readily produced.
The spray coating method is a Method wherein a substrate is moved with an ultra-atomized polymer solution sprayed onto the substrate to thereby uniformly coat the polymer solution onto the substrate. When the trigger of a spray gun is pulled, an air valve and a needle valve are simultaneously opened. The polymer solution is ejected in the form of a fine mist from a nozzle, and this polymer solution in the form of a fine mist is further ultra-atomized by air ejected from an air cap located at the end of the nozzle. A thickness-controlled polymer film is easily produced by forming the coating film of the ultra-atomized polymer solution on the substrate surface, followed by the evaporation of the solvent The thickness of the polymer thin film can be controlled on the basis of the concentration of the polymer solution, the moving speed of the substrate, and so on.
The spin coating method is a method wherein a polymer solution is added dropwise onto a substrate placed horizontally, which is then spun at a high speed to thereby uniformly coat the polymer solution onto the whole surface of the substrate through a centrifugal force. A thickness-controlled polymer film is easily produced with the scattering of the polymer solution through a centrifugal force and the evaporation of the solvent The thickness of the polymer thin film can be controlled on the basis of the revolution speed, the concentration of the polymer solution, the vapor pressure of the solvent, and so on. In the present invention, the revolution speed during spin coating is not particularly limited. If the revolution speed is too small, the solution remains on the substrate. If the revolution speed is too large, an available apparatus is restricted. Hence, in the present study, the revolution speed during spin coating is preferably 500 rpm to 10,000 rpm, more preferably 1,000 rpm to 7,000 rpm.

### <Step of forming second hydrophilic polymer layer>

In the present invention, a second hydrophilic polymer layer is directly formed on a first hydrophilic polymer layer. The term "directly" is used herein to mean that there are no physiological active substances and the like between the first hydrophilic polymer layer and the second hydrophilic polymer layer. In the present invention, the second hydrophilic polymer layer acts to prevent permeation of oxygen, oxidization of an auto-assembled membrane, and dissociation of the auto-assembled membrane. Thus, the "second hydrophilic polymer layer applied onto the first hydrophilic polymer layer" can be used in the present invention for the purpose of suppressing oxidization of the auto-assembled membrane and the dissociation thereof.
Preferably in the present invention, an aqueous solution of the hydrophilic polymer which forms the second hydrophilic polymer layer is added on the first hydrophilic polymer layer on a substrate. As a method for such addition, known methods can be used. Specific examples of such methods that can be used include an extrusion coating method, a curtain coating method, a casting method, a screen printing method, a spin coating method, a spray coating method, a slide bead coating method, a slit and spin method, a slit coating method, a dye coating method, a dip coating method, a knife coating method, a blade coating method, a flow coating method, a roll coating method, a wire-bar coating method, and a transfer printing method. As the method for forming a thin film on a substrate of the present invention, a spray coating method or a spin coating method is preferable. Further, a spin coating method is more preferable. This is because it allows a coating film having a controlled film thickness to be readily produced.
The concentration of an Application solution of a hydrophilic polymer that forms the second hydrophilic polymer layer is not particularly limited, as long as it is not problematic in terms of applicability and permeation into a layer containing a physiologically active substance. The concentration is preferably between 0.1% by weight and 5% by weight. Moreover, in terms of applicability and the control of pH, such an application solution may also comprise a surfactant, a buffer, an organic solvent, salts, etc.
As a hydrophilic polymer that forms the second hydrophilic polymer layer, a hydrophilic polymer that is nonvolatile at ordinary temperature and pressure is preferable. Such a hydrophilic polymer has a mean molecular weight, preferably of more than 350 to less than 5,000,000, more preferably of 1,200 or more to 2,000,000 or less, and most preferably of 1,200 or more to 70,000 or less. The hydrophilic polymer that forms the second hydrophilic polymer layer is preferably saccharide. Such saccharide may be either monosaccharide or polysaccharide. In the case of an n number of saccharide, n is preferably between 4 and 1,200, and more preferably between 20 and 600.
For the purpose of suppressing degradation of an self-assembled membrane immobilized on a substrate, the hydrophilic polymer that forms the second hydrophilic polymer layer preferably has a dextran skeleton or a polyethylene oxide skeleton. Any type of substituent may be used within a range that achieves the object of the present invention. Furthermore, for the purpose of suppressing degradation of the auto-assembled membrane immobilized on the substrate, it is preferably a nonionic compound having no dissociable groups. Further, the aforementioned hydrophilic polymer that forms the second hydrophilic polymer layer is preferably a compound having high affinity for water molecules. The LogP value that indicates the water/n-octanol distribution coefficient is preferably 1 or greater. The LogP value can be measured by a method described in JIS (Japanese Industrial Standard), Z7260-107 (2000), *"Bunpai keisu (1-octanol*/*mizu) no sokutei, Shinto-ho* (Measurement of Distribution Coefficient (1-octanol/water), Shaking Method)," etc.
Specific examples of the hydrophilic polymer that forms the second hydrophilic polymer layer include: polyhydric alcohols such as polyethylene glycol, collagen, gelatin, albumin, hyaluronic acid, chitin, chitosan, starch, cellulose, alginic acid, or polysaccharide such as dextran, polyethers including polyethylene oxide-polypropylene oxide condensates such as polyethylene oxide or Pluronic, compounds consisting of two or more types of residues, such as Tween 20, Tween 40, Tween 60 or Tween 80, and the derivatives and polymers of these compounds. In particular, dextran, cellulose, Tween 20, Tween 40, Tween 60 and Tween 80 are preferable. Moreover, it is preferable that these hydrophilic polymers that form the second hydrophilic polymer layer have a basic skeleton substantially idenfical to that of the used hydrophilic polymer that forms the first hydrophilic polymer layer. Herein, the term "basic skeleton" is used to mean a ring structure of sugar, for example. Even if the type of a functional group and a length are different, if such a ring structure is identical, it is considered to be substantially identical.
With regard to the content of the hydrophilic polymer that forms the second hydrophilic polymer layer on a substrate, the ratio of the mean molecular weight of the aforementioned second hydrophilic polymer to the mean molecular weight of the hydrophilic polymer that forms the first hydrophilic polymer layer is preferably between 0.005 and 0.2 in terms of the ratio of the molecular weights. If the ratio is lower than the aforementioned range, the second hydrophilic polymer is likely to be crystallized. If the ratio is greater than the aforementioned range, permeation into the first hydrophilic polymer layer becomes difficult. By controlling the content of the hydrophilic polymer that forms the second hydrophilic polymer layer with the aforementioned range, such problems are overcome, and a high effect of suppressing dissociation of the auto-assembled membrane can be obtained.
The film thickness of the second hydrophilic polymer layer in a dry state (dry conditions are as described above in the present specification) is preferably between 1 and 100 nm, more preferably between 2 and 100 mm, and particularly preferably between 5 and 100 nm. In the present invention, the total film thickness of the first hydrophilic polymer layer and the second hydrophilic polymer layer in a dry state is between 20 nm and 100 nm, and preferably between 20 nm and 50 nm.
In the present invention, immobilization of a physiologically active substance is carried out after formation of the first hydrophilic polymer layer and the second hydrophilic polymer layer. At that time, the first hydrophilic polymer layer has not chemically bound to the second hydrophilic polymer layer, and the two layers have just physically been in contact with each other. Thus, when the two layers are allowed to come into contact with (are immersed in) a solution that contains a physiologically active substance, the second hydrophilic polymer layer is detached from the substrate, and only the first hydrophilic polymer layer remains on the substrate. Herein, the second hydrophilic polymer layer can also be removed from the substrate, for example, by washing the substrate with pure water or the like, without allowing it to come into contact with a solution containing a physiologically active substance.
The physiologically active substance is immobilized on the first hydrophilic polymer layer, after the second hydrophilic polymer layer has been removed.

### <Step of immobilizing physiologically active substance>

The polymer containing a carboxyl group that forms the first hydrophilic polymer layer, which has been established on a substrate by the aforementioned method, is activated by a known method, for example, by a single use of 1-(3-Dimethylaminopropyl)-3 ethylcarbodiimide (EDC) that is water-soluble carbodiimide, or by using EDC and N-Hydroxysuccinimide (NHS) together, so that it becomes capable of immobilizing a physiologically active substance having an amino group. As described above, on the biosensor of the present invention, a physiologically active substance may be immobilized. Altematively, the present biosensor may also be provided in a state where such a physiologically active substance is not immobilized thereon. When a biosensor that is in a state where a physiologically active substance is not immobilized thereon is used, a desired physiologically active substance is immobilized on the biosensor immediately before use, and the biosensor can be then used.
As a method for activating carboxylic acid, a method described in paragraphs [0011] to [0022] of Japanese Patent Application No. 2004-238396 (JP Patent Publication (Kokai) No. 2006-58071 A) (that is, a method of activating a carboxyl group existing on the surface of a substrate using any compound selected from among a uronium salt, a phosphonium salt and a triazine derivative having a specific structure, so as to form a carboxylic acid amide group), and a method described in paragraphs [0011] to [0019] of Japanese Patent Application No. 2004-275012 (JP Patent Publication (Kokai) No. 2006-90781 A) (that is, a method of activating a carboxyl group existing on the surface of a substrate using a carbodiimide derivative or a salt thereof, then esterifying the resultant with any compound selected from among a nitrogen-containing heteroaromatic compound having a hydroxyl group, a phenol derivative having an electron attracting group, and an aromatic compound having a thiol group, and then allowing the resultant to react with amine, so as to form a carboxylic acid amide group), can preferably be used.
It is to be noted that the aforementioned uronium salt, phosphonium salt, and triazine derivative, which have a specific structure, described in Japanese Patent Application No. 2004-238396(JP Patent Publication (Kokai) No.2006-58071A), mean the uronium Salt represented by the following formula 1, the phosphonium salt represented by the following formula 2, and the triazine derivative represented by the following formula 3, respectively. 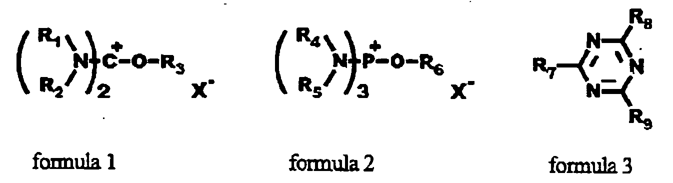 (in formula 1, each of R₁ and R₂ independently represents an alkyl group containing 1 to 6 carbon atoms, or R₁ and R₂ together form an alkylene group containing 2 to 6 carbon atoms, which forms a ring together with an N atom, R₃ represents an aromatic ring group containing 6 to 20 carbon atoms, or a hetero ring group containing at least one heteroatom, and X⁻ represents an anion; in formula 2, each of R₄ and R₅ independently represents an alkyl group containing 1 to 6 carbon atoms, or R₄ and R₅ together form an alkylene group containing 2 to 6 carbon atoms, which forms a ring together with an N atom, R₆ represents an aromatic ring group containing 6 to 20 carbon atoms, or a hetero ring group containing at least one heteroatom, and X⁻ represents an anion; and in formula 3, R₇ represents an onium group, and each of R₈ and R₉ independently represents an electron donating group.)
A physiologically active substance immobilized on the substrate of the present Invention is not particularly limited, as long as it interacts with a measurement target. Examples of such a substance may include an immune protein, an enzyme, a microorganism, nucleic acid, a low molecular weight organic compound, a nonimmune protein, an immunoglobulin-binding protein, a sugar-binding protein, a sugar chain recognizing sugar, fatty acid or fatty acid ester, and polypeptide or oligopeptide having a ligand-binding ability.
Examples of an immune protein may include an antibody whose antigen is a measurement target, and a hapten. Examples of such an antibody may include various immunoglobulins such as IgG, IgM, IgA, IgE or IgD. More specifically, when a measurement target is human serum albumin, an anti-human serum albumin antibody can be used as an antibody. When an antigen is an agricultural chemical, pesticide, methicillin-resistant *Staphylococcus aureus,* antibiotic, narcotic drug, cocaine, heroin, crack or the like, there can be used, for example, an anti-atrazine antibody, anti-kanamycin antibody, anti-metamphetamine antibody, or antibodies against O antigens 26, 86, 55, 111 and 157 among enteropathogenic *Escherichia coli.*
An enzyme used as a physiologically active substance herein is not particularly limited, as long as it exhibits an activity to a measurement target or substance metabolized from the measurement target Various enzymes such as oxidoreductase, hydrolase, isomerase, lyase or synthetase can be used. More specifically, when a measurement target is glucose, glucose oxidase is used, and when a measurement target is cholesterol, cholesterol oxidase is used. Moreover, when a measurement target is an agricultural chemical, pesticide, methicillin-resistant *Staphylococcus aureus,* antibiotic, narcotic drug, cocaine, heroin, crack or the like, enzymes such as acetylcholine esterase, catecholamine esterase, noradrenalin esterase or dopamine esterase, which show a specific reaction with a substance metabolized from the above measurement target, can be used.
A microorganism used as a physiologically active substance herein is not particularly limited, and various microorganisms such as *Escherichia coli* can be used. As nucleic acid, those complementarily hybridizing with nucleic acid as a measurement target can be used. Either DNA (including cDNA) or RNA can be used as nucleic acid. The type of DNA is not particularly limited, and any of native DNA, recombinant DNA produced by gene recombination and chemically synthesized DNA may be used.
As a low molecular weight organic compound, any given compound that can be synthesized by a common method of synthesizing an organic compound can be used.
A nonimmune protein used herein is not particularly limited, and examples of such a nonimmune protein may include avidin (streptoavidin), biotin, and a receptor. Examples of an immunoglobulin-binding protein used herein may include protein A, protein G, and a rheumatoid factor (RF). As a sugar-binding protein, for example, lectin is used. Examples of fatty acid or fatty acid ester may include stearic acid, arachidic acid, behenic acid, ethyl stearate, ethyl arachidate, and ethyl behenate.
As a physiologically active substance to be immobilized on a substrate in the present invention, a ligand that forms a bond with a tag molecule (a molecule that forms a specific affinity with a specific ligand) at Kd (dissociation constant) of 10⁻¹² M or less is preferable. Specifically, protein A, protein G, avidins, calmodulin, and an antibody are preferable. A ligand that forms a bond with such a tag molecule at Kd of 10⁻¹² M or less is used as a binding group, and it becomes possible to immobilize a specific physiologically active substance modified with the tag molecule on a substrate, while suppressing a decrease in the activity of the aforementioned specific physiologically active substance. Examples of a combination of a tag molecule with a ligand include a biotin/a biotin-binding protein, a digoxigenin/a digoxigenin antibody, and a D-Ala-D-Ala derivative/a vancomycin trimer derivative described in SCIENCE, 280, 708-711 (1998). Specific examples of a biotin-binding protein include avidins (avidin, streptavidin, NeutrAvidin, or a modified compound thereof, etc.) Such avidins are particularly preferable in terms of stability on a substrate and a low Kd value.
It is preferred that a solution which contains the physiologically active substance is an aqueous solution. The concentration is preferably between 0.001 mg/ml and 10 mg/ml, and more preferably between 0,002 mg/ml and 5 mg/ml, and particularly preferably between 0.005 mg/ml and 1 mg/ml. In some cases, an additive such as a surfactant or a pH adjusting agent may be used.
A substrate-to which a physiologically active substance is immobilized as described above can be used to detect and/or measure a substance which interacts with the physiologically active substance.
In the present invention, it is preferable to detect and/or measure an interaction between a physiologically active substance immobilized on the substrate and a test substance by a nonelectric chemical method. Examples of a non-electrochemical method may include a surface plasmon resonance (SPR) measurement technique, a quartz crystal microbalance (QCM) measurement technique, and a measurement technique that uses functional surfaces ranging from gold colloid particles to ultra-fine particles.
In a preferred embodiment of the present invention, the substrate of the present invention can be used as a biosensor for surface plasmon resonance which is characterized in that it comprises a metal film placed on a transparent substrate.
A biosensor for surface plasmon resonance is a biosensor used for a surface plasmon resonance biosensor, meaning a member comprising a portion for transmitting and reflecting light emitted from the sensor and a portion for immobilizing a physiologically active substance. It may be fixed to the main body of the sensor or may be detachable.
The surface plasmon resonance phenomenon occurs due to the fact that the intensity of monochromatic light reflected from the border between an optically transparent substance such as glass and a metal thin film layer depends on the refractive index of a sample located on the outgoing side of the metal. Accordingly, the sample can be analyzed by measuring the intensity of reflected monochromatic light.
A device using a system known as the Kretschmarm configuration is an example of a surface plasmon measurement device for analyzing the properties of a substance to be measured using a phenomenon whereby a surface plasmon is excited with a lightwave (for example, Japanese Patent Laid-Open No. 6-167443). The surface plasmon measurement device using the above system basically comprises a dielectric block formed in a prism state, a metal film that is formed on a face of the dielectric block and comes into contact with a measured substance such as a sample solution, a light source for generating a light beam, an optical system for allowing the above light beam to enter the dielectric block at various angles so that total reflection conditions can be obtained at the interface between the dielectric block and the metal film, and a light-detecting means for detecting the state of surface plasmon resonance, that is, the state of attenuated total reflection, by measuring the intensity of the light beam totally reflected at the above interface.
In order to achieve various incident angles as described above, a relatively thin light beam may be caused to enter the above interface while changing an incident angle. Otherwise, a relatively thick light beam may be caused to enter the above interface in a state of convergent light or divergent light, so that the light beam contains components that have entered therein at various angles. In the former case, the light beam whose reflection angle changes depending on the change of the incident angle of the entered light beam can be detected with a small photodetector moving in synchronization with the change of the above reflection angle, or it can also be detected with an area sensor extending along the direction in which the reflection angle is changed. In the latter case, the light beam can be detected with an area sensor extending to a direction capable of receiving all the light beams reflected at various reflection angles.
With regard to a surface plasmon measurement device with the above structure, if a light beam is allowed to enter the metal film at a specific incident angle greater than or equal to a total reflection angle, then an evanescent wave having an electric distribution appears in a measured substance that is in contact with the metal film, and a surface plasmon is excited by this evanescent wave at the interface between the metal film and the measured substance. When the wave vector of the evanescent light is the same as that of a surface plasmon and thus their wave numbers match, they are in a resonance state, and light energy transfers to the surface plasmon. Accordingly, the intensity of totally reflected light is sharply decreased at the interface between the dielectric block and the metal film. This decrease in light intensity is generally detected as a dark line by the above light-detecting means. The above resonance takes place only when the incident beam is p-polarized light. Accordingly, it is necessary to set the light beam in advance such that it enters as p-polarized light.
If the wave number of a surface plasmon is determined from an incident angle causing the attenuated total reflection (AIR), that is, an attenuated total reflection angle (θSP), the dielectric constant of a measured substance can be determined. As described in Japanese Patent Laid-Open No. 11-326194, a light-detecting means in the form of an array is considered to be used for the above type of surface plasmon measurement device in order to measure the attenuated total reflection angle (θSP) with high precision and in a large dynamic range. This light-detecting means comprises multiple photo acceptance units that are arranged in a certain direction, that is, a direction in which different photo acceptance units receive the components of light beams that are totally reflected at various reflection angles at the above interface.
In the above case, there is established a differentiating means for differentiating a photodetection signal outputted from each photo acceptance unit in the above array-form light-detecting means with regard to the direction in which the photo acceptance unit is arranged. An attenuated total reflection angle (θSP) is then specified based on the derivative value outputted from the differentiating means, so that properties associated with the refractive index of a measured substance are determined in many cases. In addition, a leaking mode measurement device described in "Bunko Kenkyu (Spectral Studies)" Vol. 47, No. 1 (1998), pp. 21 to 23 and 26 to 27 has also been known as an example of measurement devices similar to the above-described device using attenuated total reflection (ATR). This leaking mode measurement device basically comprises a dielectric block formed in a prism state, a clad layer that is formed on a face of the dielectric block, a light wave guide layer that is formed on the clad layer and comes into contact with a sample solution, a light source for generating a light beam, an optical system for allowing the above light beam to enter the dielectric block at various angles so that total reflection conditions can be obtained at the interface between the dielectric block and the clad layer, and a light-detecting means for detecting the excitation state of waveguide mode, that is, the state of attenuated total reflection, by measuring the intensity of the light beam totally reflected at the above interface.
In the leaking mode measurement device with the above structure, if a light beam is caused to enter the clad layer via the dielectric block at an incident angle greater than or equal to a total reflection angle, only light having a specific wave number that has entered at a specific incident angle is transmitted in a waveguide mode into the light wave guide layer, after the light beam has penetrated the clad layer. Thus, when the waveguide mode is excited, almost all forms of incident light are taken into the light wave guide layer, and thereby the state of attenuated total reflection occurs, in which the intensity of the totally reflected light is sharply decreased at the above interface.
Since the wave number of a waveguide light depends on the refractive index of a measured substance placed on the light wave guide layer, the refractive index of the measurement substance or the properties of the measured substance associated therewith can be analyzed by determining the above specific incident angle causing the attenuated total reflection.
In this leaking mode measurement device also, the above-described array-form light-detecting means can be used to detect the position of a dark line generated in a reflected light due to attenuated total reflection. In addition, the above-described differentiating means can also be applied in combination with the above means.
The above-described surface plasmon measurement device or leaking mode measurement device may be used in random screening to discover a specific substance binding to a desired sensing substance in the field of research for development of new drugs or the like. In this case, a sensing substance is immobilized as the above-described measured substance on the above thin film layer (which is a metal film in the case of a surface plasmon measurement device, and is a clad layer and a light guide wave layer in the case of a leaking mode measurement device), and a sample solution obtained by dissolving various types of test substance in a solvent is added to the sensing substance. Thereafter, the above-described attenuated total reflection angle (θSP) is measured periodically when a certain period of time has elapsed.
If the test substance contained in the sample solution is bound to the sensing substance, the refractive index of the sensing substance is changed by this binding over time. Accordingly, the above attenuated total reflection angle (θSP) is measured periodically after the elapse of a certain time, and it is determined whether or not a change has occurred in the above attenuated total reflection angle (θSP), so that a binding state between the test substance and the sensing substance is measured. Based on the results, it can be determined whether or not the test substance is a specific substance binding to the sensing substance. Examples of such a combination between a specific substance and a sensing substance may include an antigen and an antibody, and an antibody and an antibody. More specifically, a rabbit anti-human IgG antibody is immobilized as a sensing substance on the surface of a thin film layer, and a human IgG antibody is used as a specific substance.
It is to be noted that in order to measure a binding state between a test substance and a sensing substance, it is not always necessary to detect the angle itself of an attenuated total reflection angle (θSP). For example, a sample solution may be added to a sensing substance, and the amount of an attenuated total reflection angle (θSP) changed thereby may be measured, so that the binding state can be measured based on the magnitude by which the angle has changed. When the above-described array-form light-detecting means and differentiating means are applied to a measurement device using attenuated total reflection, the amount by which a derivative value has changed reflects the amount by which the attenuated total reflection angle (θSP) has changed. Accordingly, based on the amount by which the derivative value has changed, a binding state between a sensing substance and a test substance can be measured (Japanese Patent Application No. 2000-398309 filed by the present applicant). In a measuring method and a measurement device using such attenuated total reflection, a sample solution consisting of a solvent and a test substance is added dropwise to a cup- or petri dish-shaped measurement chip wherein a sensing substance is immobilized on a thin film layer previously formed at the bottom, and then, the above-described amount by which an attenuated total reflection angle (θSP) has changed is measured.
Moreover, Japanese Patent Laid-Open No. 2001-330560 describes a measurement device using attenuated total reflection, which involves successively measuring multiple measurement chips mounted on a turntable or the like, so as to measure many samples in a short time.
When the biosensor of the present invention is used in surface plasmon resonance analysis, it can be applied as a part of various surface plasmon measurement devices described above.
Further, the substrate of the present invention can be used as a biosensor, which has a waveguide structure on the surface of a substrate, for example, and which detects refractive index changes using such a waveguide. The measurement technology of detecting refractive index changes using a waveguide is a technology of detecting an effective refractive index change of a medium adjacent to the waveguide by optical change. The structure of a biosensor of this system is described in column 6, line 31 to column 7, line 47, and Figures 9A and 9B of US Patent No. 6,829,073.
The present invention will be further specifically described in the following examples. However, the examples are not intended to limit the scope of the present invention.

### Examples

### Example 1

### (1) Preparation of substrate

An aqueous solution of 1 mM 6-Amino-1-octanethiol, hydrochloride (manufactured by Dojindo Laboratories) was prepared. This solution was designated as Solution A.
Next, a gold thin film was formed on the upper surface of a plastic prism obtained by injection-molding ZEONEX (manufactured by Zeon Corp) according to a method described below
The prism was secured to a substrate holder of a sputtering apparatus, and a vacuum (base pressure of 1×10⁻³ Pa or less) was drawn therein. Thereafter, Ar gas was introduced (1 Pa) into the apparatus, and RF power (0.5 kW) was applied to the substrate holder for approximately 9 minutes with the substrate holder rotated (20 rpm), so as to plasma-treat the prism surface. Next, Ar gas was stopped, and a vacuum was drawn therein. Ar gas was introduced (0.5 Pa) again into the apparatus, and DC power (0.2 kW) was applied to a 8-inch Cr target for approximately 30 seconds with the substrate holder rotated (10 to 40 rpm), so as to form a 2-mn Cr thin film thereon. Subsequently, Ar gas was stopped, and a vacuum was drawn again. Ar gas was introduced (0.5 Pa) again into the apparatus, and DC power (1 kW) was applied to a 8-inch Au target for approximately 50 seconds with the substrate holder rotated (20 rpm), so as to form an approximately 50-nm Au thin film thereon.
The thus obtained plastic prism, on which the Au thin film had been formed, was immersed in the SolutionA at 40°C for 1 hour, and it was then washed five times with ultrapure water.

### (2) Active esterification of CMD (carboxymethyl dextran)

After dissolution of 20 g of a solution of 1% by weight of CMD (manufactured by Meito Sangyo; molecular weight of 1,000,000), 20 ml of EDC (1-Ethyl-3-[3-Dimethylaminopropyllcabodiimide Hydrochloride) in each of the concentrations as shown in Table 1 was added thereto, and the obtained mixture was then stirred at room temperature for 1 hour.

### (3) Binding reaction of CMD to substrate

Onto the substrate prepared in (1) above, 1ml of the mixed solution of CMD and EDC prepared in (2) above was added dropwise, and it was then spin-coated at 1000 rpm for 45 seconds. The resultant was then reacted by leaving it at rest at room temperature for 15 minutes, so as to immobilize a thin film of carboxymethyl dextran on the substrate having an amino group. The substrate was immersed in a 1N NaOH aqueous solution for 30 minutes, and it was then washed 5 times with ultrapure water to obtain CMD film-coated sensor sticks (samples 1 to 5). The thickness of each CMD film was measured using an ellipsometer. The results are shown in Table 1.

### Example 2

0.1 ml of an aqueous solution of 2%-by-weight dextran (manufactured by Meito Sangyo Co., Ltd.; mean molecular weight: 100,000) was casted on each of the CMD films of samples 2, 4 and 5 produced in Example 1, and it was then spin-coated at 500 rpm for 45 seconds, so as to obtain sensor sticks (samples 2, 4 and 5) coated with dextran. The thickness of the dextran film was calculated based on a difference between before and after application by such ellipsometer measurement. The results are shown in Table 1.

### Example 3

Example 3 relates to preservation of the sensor samples obtained in Examples 1 and 2. Each of the samples produced in Examples 1 and 2 (samples 1 to 5) was placed in an aluminum bag, and the bag was then filled with nitrogen. Thereafter, the bag was hermetically sealed, and it was then left at 50°C for 2 weeks. Thereafter, the sample was immersed in a 1 N NaOH aqueous solution for 15 minutes, and it was then washed with ultrapure water 5 times. The resultant was measured with an ellipsometer, and the film thickness of the remaining CMD was measured.
The residual amount of CMD and the residual rate of each sample are shown in Table 1. The larger the amount of CMD immobilized, the more the physiologically active substance that can preferably be immobilized. It is found that the sensor stick of the present invention having a total film thickness of 20 nm or greater has a high residual polymer rate, and that it is able to immobilize a large amount of physiologically active substance even if a certain period of time is forced to pass.

**Table 1:**

| | EDC concentration (mM) | Hydrophilic polymer 1/film thickness (nm) | Hydrophilic polymer 2/film thickness (nm) | Total film thickness of hydrophilic polymers (nm) | Film thickness of hydrophilic polymer 1 (nm) after time has passed | Residual rate of hydrophilic polymer 1 (%) | Remarks |
|---|---|---|---|---|---|---|---|
| 1 | 8 | CMD/13.7 | - | 13.7 | 4.7 | 34 | Comparative example |
| 2 | 8 | CMD/13.7 | Dex/10.0 | 23.7 | 10.3 | 75 | The present Invention |
| 3 | 20 | CMD/29.0 | - | 29.0 | 26.0 | 90 | Reference example |
| 4 | 15 | CMD/18.7 | Dex/10.0 | 28.7 | 18.0 | 96 | The present invention |
| 5 | 20 | CMD/29.5 | Dex10.0 | 39.5 | 29.5 | 100 | The present invention |

| | | | | | | | |
|---|---|---|---|---|---|---|---|
| CMD: carboxymethyl dextran Dex: dextran Time passed: 50°C, 2 weeks | | | | | | | |

## Claims

1. A biosensor wherein a first hydrophilic polymer layer is immobilized on a self-assembled membrane applied onto the surface of a metal substrate, a second hydrophilic polymer layer is applied onto the first hydrophilic polymer layer, and the total film thickness of the first hydrophilic polymer layer and the second hydrophilic polymer layer in a dry state is between 20 nm and 100 nm.

2. The biosensor according to claim 1, wherein the hydrophilic polymer that forms the first hydrophilic polymer layer and the second hydrophilic polymer layer is a polysaccharide.

3. The biosensor according to claim 1, wherein the self-assembled membrane is formed by a compound having a mercapto group.

4. The biosensor according to claim 1, wherein the self-assembled membrane is formed by a compound represented by the following formula A-1:
**HS(CH₂)ₙX** A-1
wherein n represents an integer between 3 and 20, and X represents a functional group for binding a hydrophilic polymer.

5. The biosensor according to claim 1, wherein the self-assembled membrane is formed by coating the surface of the metal substrate with an aqueous solution of a compound having a straight-chain alkyl group containing 4 to 10 carbon atoms.

6. The biosensor according to claim 1, which is used in nonelectrochemieal detection.

7. The biosensor according to claim 1, which is used in surface plasmon resonance analysis.

## Patentansprüche

1. Biosensor, worin eine erste hydrophile Polymerschicht auf einer selbst zusammengebauten Membran, die auf die Oberfläche eines Metallsubstrates aufgetragen ist, immobilisiert ist, eine zweite hydrophile Polymerschicht auf die erste hydrophile Polymerschicht aufgetragen ist und die Gesamtfilmdicke der ersten hydrophilen Polymerschicht und der zweiten hydrophilen Polymerschicht in einem Trockenzustand zwischen 20 und 100 nm ist.

2. Biosensor nach Anspruch 1, worin das hydrophile Polymer, das die erste hydrophile Polymerschicht und die zweite hydrophile Polymerschicht bildet, ein Polysaccharid ist.

3. Biosensor nach Anspruch 1, worin die selbst zusammengebaute Membran durch eine Verbindung mit einer Mercaptogruppe gebildet ist.

4. Biosensor nach Anspruch 1, worin die selbst zusammengebaute Membran durch eine Verbindung gebildet ist, dargestellt durch die folgende Formel A-1:
HS(CH₂)ₙX A-1
worin n eine ganze Zahl zwischen 3 und 20 ist und X eine funktionelle Gruppe zum Binden eines hydrophilen Polymers ist.

5. Biosensor nach Anspruch 1, worin die selbst zusammengebaute Membran durch Beschichten der Oberfläche des Metallsubstrates mit einer wässrigen Lösung aus einer Verbindung mit einer geradkettigen Alkylgruppe mit 4 bis 10 Kohlenstoffatomen gebildet ist.

6. Biosensor nach Anspruch 1, der bei der nichtelektrochemischen Detektion verwendet wird.

7. Biosensor nach Anspruch 1, der bei der Oberflächenplasmaresonanzanalyse verwendet wird.

## Revendications

1. Biocapteur dans lequel une première couche de polymère hydrophile est immobilisée sur une membrane auto-assemblée appliquée sur la surface d'un substrat métallique, une seconde couche de polymère hydrophile est appliquée sur la première couche de polymère hydrophile, et l'épaisseur totale de film de la première couche de polymère hydrophile et de la seconde couche de polymère hydrophile à l'état sec est entre 20 nm et 100 nm.

2. Biocapteur selon la revendication 1, dans lequel le polymère hydrophile qui forme la première couche de polymère hydrophile et la seconde couche de polymère hydrophile est un polysaccharide.

3. Biocapteur selon la revendication 1, dans lequel la membrane auto-assemblée est formée par un composé ayant un groupe mercapto.

4. Biocapteur selon la revendication 1, dans lequel la membrane auto-assemblée est formée par un composé représenté par la formule A-1 suivante :
HS(CH₂)nX A-1
dans laquelle n représente un nombre entier compris entre 3 et 20, et X représente un groupe fonctionnel pour lier un polymère hydrophile.

5. Biocapteur selon la revendication 1, dans lequel la membrane auto-assemblée est formée par revêtement de la surface du substrat métallique avec une solution aqueuse d'un composé ayant un groupe alkyle à chaîne linéaire contenant 4 à 10 atomes de carbone.

6. Biocapteur selon la revendication 1, qui est utilisé dans une détection non électrochimique.

7. Biocapteur selon la revendication 1, qui est utilisé dans une analyse par résonance plasmonique de surface.
